(19) Europäisches Patentamt
European Patent Office
Office européen des brevets

(11) EP 1 305 357 B1

(12) EUROPÄISCHE PATENTSCHRIFT

(45) Veröffentlichungstag und Bekanntmachung des Hinweises auf die Patenterteilung:
**13.10.2004 Patentblatt 2004/42**

(21) Anmeldenummer: **01917106.5**

(22) Anmeldetag: **22.03.2001**

(51) Int Cl.[7]: **C08G 77/388**, D06M 15/643, A61K 7/06, C08L 83/08, C09D 183/08

(86) Internationale Anmeldenummer:
**PCT/EP2001/003291**

(87) Internationale Veröffentlichungsnummer:
**WO 2002/010254 (07.02.2002 Gazette 2002/06)**

(54) **WÄSSRIGE ZUSAMMENSETZUNGEN**
AQUEOUS COMPOSITIONS
COMPOSITIONS AQUEUSES

(84) Benannte Vertragsstaaten:
**DE FR GB**

(30) Priorität: **27.07.2000 DE 10036677**

(43) Veröffentlichungstag der Anmeldung:
**02.05.2003 Patentblatt 2003/18**

(73) Patentinhaber: **Wacker-Chemie GmbH**
**81737 München (DE)**

(72) Erfinder:
- **HERZIG, Christian**
**83329 Waging am See (DE)**
- **SCHATTENMANN, Wolfgang**
**84489 Burghausen (DE)**

(74) Vertreter: **Deffner-Lehner, Maria, Dr. et al**
**Wacker-Chemie GmbH,**
**Hanns-Seidel-Platz 4**
**81737 München (DE)**

(56) Entgegenhaltungen:
**US-A- 4 833 225** **US-A- 5 707 434**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 305 357 B1

Printed by Jouve, 75001 PARIS (FR)

**Beschreibung**

In US 4,101,272 (Commonwealth Scientific and Industrial Research Organization, ausgegeben am 18. Juli 1978) wird ein Verfahren zur Wollbehandlung beschrieben, bei dem Epoxysiloxane und Amine als separate Stoffe auf die Wolle aufgebracht werden und in situ reagieren unter Bildung von vernetzten nichtlöslichen Strukturen.

US 4,833,225 (Goldschmidt, ausgegeben am 23 Mai 1989) offenbart polyquaternäre Polysiloxanpolymere der Blockstruktur $(AB)_nA$, die durch Umsetzung von $\alpha,\omega$-Epoxysiloxanen mit di-tertiären Diaminen in Gegenwart von Säuren erhalten werden. Die Blockcopolymere enthalten quaternäre Stickstoffatome.

In US 5,807,956 (OSi Specialties, Inc., ausgegeben am 15. September 1998) sind Blockcopolymere mit $(AB)_nA$-Struktur, die Polyalkylenoxidketten enthalten, beschrieben. Die Herstellung erfolgt durch Umsetzung von $\alpha$, $\omega$-Epoxysiloxanen mit $\alpha,\omega$-Aminoalkylpolyethern, wobei wegen der sehr schlechten gegenseitigen Löslichkeit der Edukte größere Mengen an organischen Lösungsmitteln erforderlich sind um ausreichende Kompatibilität zu erzielen.

Aus US-A 6,001,422 (Shin-Etsu Chemical Co., ausgegeben am 14.12.1999) sind Aminoalkylgruppen enthaltende Diorganopolysiloxane und deren Verwendung in wäßrigen Formulierungen bekannt, deren basische Stickstoffatome durch - $CH_2$-$CH_2$-Gruppen getrennt sind. Die beschriebenen Aminosiloxane haben Aminequivalentwerte von 5 000 bis 100 000 g/Mol, was einem Gehalt an Aminstickstoff von 0,014 bis 0,28 Gew.-% entspricht. Es bestand die Aufgabe wäßrige Zusammensetzungen von Organosiliciumverbindungen, die organische Reste mit basischem Stickstoff aufweisen, bereitzustellen, wobei die Organosiliciumverbindungen insbesondere in Wasser löslich oder selbstdispergierend sind, und dabei insbesondere einen geringeren Gehalt an basischem Stickstoff aufweisen als bisher. Die Aufgabe wird durch die Erfindung gelöst.

Gegenstand der Erfindung sind wässrige Zusammensetzungen enthaltend

(1) Ammoniumgruppen aufweisende Organosiliciumverbindungen, die pro Molekül mindestens eine Gruppierung der allgemeinen Formel

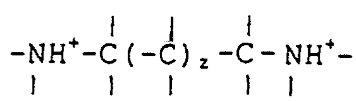

wobei z eine ganze Zahl von 1 bis 20 bedeutet, enthalten,
(2) Säureanionen, deren korrespondierende Säuren einen $pk_s$-Wert von größer als 0, vorzugsweise größer als 2, bevorzugt größer als 3, haben,
(3) Wasser,
gegebenenfalls
(4) mit Wasser mischbare Lösemittel
und gegebenenfalls
(5) Emulgatoren.

Die wässrigen Zusammensetzungen sind vorzugsweise homogene wässrige Lösungen, wässrige Mikroemulsionen und wässrige Emulsionen, wobei homogene wässrige Lösungen bevorzugt sind.

Die erfindungsgemäßen wässrigen Zusammensetzungen werden hergestellt indem die erfindungsgemäßen (1) Ammoniumgruppen aufweisenden Organosiliciumverbindungen zusammen mit ihren (2) Säureanionen mit (3) Wasser, gegebenenfalls (4) mit Wasser mischbare Lösemittel und gegebenenfalls (5) Emulgatoren gemischt werden.

Die erfindungsgemäßen (1) Ammoniumgruppen aufweisenden Organosiliciumverbindungen zusammen mit ihren (2) Säureanionen werden vorzugsweise erhalten indem die zu (1) korrespondierenden Aminogruppen aufweisenden Organosiliciumverbindungen mit zu (2) korrespondierenden Säuren umgesetzt werden.

Als erfindungsgemäße Ammoniumgruppen aufweisenden Organosiliciumverbindungen werden vorzugsweise solche eingesetzt, die

(a) mindestens eine Struktureinheit Y der allgemeinen Formel

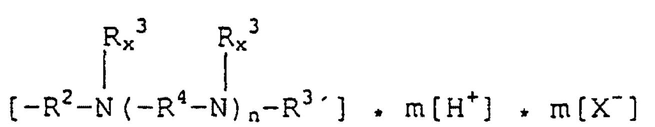 (I)

wobei $R^2$ ein zweiwertiger organischer Rest ist,

$R^3$ ein Wasserstoffatom oder einen einwertigen Kohlenwasserstoffrest mit 1 bis 60 Kohlenstoffatomen je Rest, der durch ein oder mehrere separate Heteroatome ausgewählt aus der Gruppe der Stickstoff-, Sauerstoff-, Schwefel- oder Halogenatome unterbrochen oder substituiert sein kann, bedeutet,

$R^{3'}$ die Bedeutung von $R^3$ hat, vorzugsweise ein Wasserstoffatom oder ein einwertiger Kohlenwasserstoffrest mit 1 bis 12 Kohlenstoffatomen je Rest ist,

$R^4$ ein zweiwertiger Kohlenwasserstoffrest mit 3 bis 10 Kohlenstoffatomen je Rest, bevorzugt 3 bis 10 Kohlenstoffatomen je Rest, ist,

n 0 oder eine ganze Zahl von 1 bis 10 ist

m eine ganze Zahl von 2 bis zur Gesamtanzahl der Stickstoffatome in (I), vorzugsweise eine ganze Zahl von 2 bis zur Summe aus n+1 und der Gesamtanzahl aller in den Resten $R^3$ gegebenenfalls enthaltenen basischen Stickstoffatome, ist und

x gleich oder verschieden ist und 0 oder 1 ist,

$X^-$ ein Säureanion ist, deren korrespondierende Säure einen $pK_s$-Wert von größer als 0, vorzugsweise größer als 2, bevorzugt größer als 3, hat,

mit der Maßgabe, daß die Struktureinheit der Formel (I) mindestens eine Gruppierung aus zwei protonierten Stickstoffatomen enthält, die über mindestens drei Kohlenstoffatome miteinander verbunden sind,

(b) mindestens eine Siloxaneinheit der allgemeinen Formel

$$R_a(R^1O)_bSiO_{\frac{4-(a+b)}{2}} \qquad \text{(II)}$$

wobei R gleich oder verschieden ist und einen einwertigen gegebenenfalls halogenierten Kohlenwasserstoffrest mit 1

bis 18 Kohlenstoffatomen je Rest bedeutet,

$R^1$ gleich oder verschieden ist und einen einwertigen

Kohlenwasserstoffrest mit 1 bis 8 Kohlenstoffatomen je Rest bedeutet,

a 0, 1, 2 oder 3 ist,

b 0, 1, 2 oder 3 ist,

mit der Maßgabe, dass die Summe a+b≤3 ist,

und

(c) mindestens eine Siloxaneinheit der allgemeinen Formel

$$R_a(R^1O)_b\overset{|}{Si}O_{\frac{3-(a+b)}{2}} \qquad \text{(III)}$$

wobei R, $R^1$, a und b die oben dafür angegebene Bedeutung haben,

mit der Maßgabe, dass die Summe a+b≤2 ist und

dass die Siloxaneinheit der Formel (III) über die Si-Atome mit der Struktureinheit der Formel (I) über die Reste $R^2$

verbunden ist,

und gegebenenfalls

(d) mindestens eine Struktureinheit Y' der allgemeinen Formel

$$[-R^2-\overset{R_x^3}{\overset{|}{N}}(-R^4-\overset{R_x^3}{\overset{|}{N}})_n-R^2-] * m[H^+] * m[X^-] \quad \text{(I')}$$

wobei $R^2$, $R^3$, $R^4$, n, m, x und $X^-$ die oben dafür angegebene Bedeutung haben,

mit der Maßgabe, daß die Struktureinheit der Formel (I') mindestens eine Gruppierung aus zwei protonierten Stickstoffatomen enthält, die über mindestens drei Kohlenstoffatome miteinander verbunden sind, und

(e) mindestens zwei Siloxaneinheiten der allgemeinen Formel

$$\begin{array}{c} | \\ R_a(R^1O)_bSiO_{\frac{3-(a+b)}{2}} \end{array} \qquad (III')$$

wobei R, $R^1$, a und b die oben dafür angegebene Bedeutung haben,
mit der Maßgabe, dass die Summe a+b≤2 ist und
dass die Siloxaneinheiten der Formel (III') über die Siatome mit der Struktureinheit der Formel (I') über die Reste $R^2$ verbunden sind.

enthalten.

Als erfindungsgemäße Ammoniumgruppen aufweisende Organosiliciumverbindungen werden bevorzugt solche eingesetzt, die

(a') mindestens eine Siloxaneinheit der allgemeinen Formel

$$YR_a(R^1O)_bSiO_{\frac{4-(a+b)}{2}} \qquad (IV)$$

wobei Y ein organischer Rest der Formel (I) bedeutet und R, $R^1$, a und b die oben dafür angegebene Bedeutung haben, mit der Maßgabe, dass die Summe a+b≤2 ist,
(b) mindestens eine Siloxaneinheit der allgemeinen Formel

$$R_a(R^1O)_bSiO_{\frac{4-(a+b)}{2}} \qquad (II)$$

wobei R, $R^1$, a und b die oben dafür angegebene Bedeutung haben, mit der Maßgabe, dass die Summe a+b≤3 ist
und gegebenenfalls
(d') mindestens eine Brückeneinheit der allgemeinen Formel

$$\begin{array}{c} R_a(R^1O)_bSiO_{\frac{3-(a+b)}{2}} \\ | \\ Y' \\ | \\ R_a(R^1O)_bSiO_{\frac{3-(a+b)}{2}} \end{array} \qquad (V)$$

wobei Y' ein organischer Rest der Formel (I') ist und
R, $R^1$, a und b die oben dafür angegebene Bedeutung haben, mit der Maßgabe, dass die Summe a+b≤2 ist,

enthalten.

Vorzugsweise beträgt der Gehalt an Ammoniumstickstoff 0,3 bis 5,0 Gew.-%, bevorzugt 0,5 bis 5,0 Gew.-%, jeweils bezogen auf das Gesamtgewicht der Ammoniumgruppen aufweisenden Organosiliciumverbindungen.

Die erfindungsgemäßen Ammoniumgruppen aufweisenden Organosiliciumverbindungen haben vorzugsweise einen Viskosität von 50 - 5.000.000 mPa.s bei 25°C, bevorzugt 100 - 100.000 mPa.s bei 25°C.

Die erfindungsgemäßen Ammoniumgruppen aufweisenden Organosiliciumverbindungen können hergestellt werden durch Umsetzung von (Poly)aminen mit Epoxygruppen aufweisenden Organosiliciumverbindungen.
Enthält das (Poly)amin nur ein Stickstoff-gebundenes Wasserstoffatom findet eine Monoaddition statt.
Die Monoaddition von (Poly)aminen mit einem N-gebundenen Wasserstoffatom mit Epoxygruppen aufweisenden Organosiliciumverbindungen ist bekannt und beispielsweise in US-A 3,389,160 (Union Carbide Corporation) beschrieben.
Enthält das (Poly)amin mindestens zwei Stickstoff-gebundene Wasserstoffatome findet eine Polyaddition statt.

Die Protonierung der Stickstoffatome erfolgt dann durch Umsetzung mit Säuren.

Werden die erfindungsgemäßen Ammoniumgruppen aufweisenden Organosiliciumverbindungen durch die oben genannte Polyaddition hergestellt, enthalten sie dann zusätzlich zu den Einheiten (a), (b) und (c) bzw. (a') und (b) die Einheiten (d) und (e), d.h. die Brückeneinheiten (d').

Als erfindungsgemäße Ammoniumgruppen aufweisende Organosiliciumverbindungen können auch solche eingesetzt werden, die hergestellt werden durch Umsetzung von Chloralkylalkoxysilanen, wie Chloralkyldimethoxymethylsilanen, mit (Poly)aminen, wie Hexamethylendiamin, und Equilibrierung der so erhaltenen Aminoalkylalkoxysilane, wie Aminoalkyldimethoxymethylsilane, oder deren Hydrolyse- und/oder Kondensationsprodukte, mit Organopolysiloxanen, wie linearen, endständige Triorganosiloxygruppen aufweisenden Organopolysiloxanen, linearen, endständige Hydroxylgruppen aufweisenden Organopolysiloxanen, cyclischen Organopolysiloxanen oder Mischpolymerisaten aus Diorganosiloxan- und Monoorganosiloxaneinheiten.

Die Protonierung der Stickstoffatome erfolgt dann durch Umsetzung mit Säuren.

Solche aminofunktionellen Organosiliciumverbindungen sowie deren Herstellung sind bekannt und beispielsweise beschrieben in US-A 2,971,864 (Dow Corning Corporation, ausgegeben am 14.02.1961) und J.L.Speier et al., J. Org. Chem. 36, 3120 (1971).

Beispiele für Reste R sind Alkylreste, wie der Methyl-, Ethyl-, n-Propyl-, iso-Propyl-, n-Butyl-, iso-Butyl-, tert. -Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest, Cycloalkylreste, wie Cyclopentyl-, Cyclohexyl-, Cycloheptylund Methylcyclohexylreste, Arylreste, wie der Phenyl-, Naphthyl-, Anthryl- und Phenanthrylrest, Alkarylreste, wie o-, m-, p-Tolylreste, Xylylreste und Ethylphenylreste, und Aralkylreste, wie der Benzylrest, der $\alpha$- und der $\beta$-Phenylethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m- und p-Chlorphenylrest.

Bevorzugt ist R ein Methylrest.

Beispiele für Reste $R^1$ sind Alkylreste, wie der Methyl-, Ethyl-, n-Propyl-, iso-Propyl-, n-Butyl-, iso-Butyl-, tert. -Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest.

Vorzugsweise ist $R^2$ ein organischer Rest ausgewählt aus der Gruppe von

$$(Si)\text{-}(R^5)_t\text{-}CR^6(OH)\text{-}CR^6{}_2\text{-}\ (N) \qquad (VI),$$

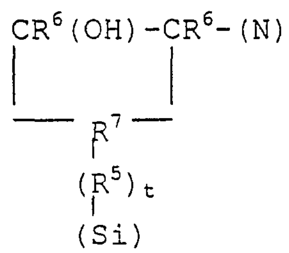

. (VII)

und

ein zweiwertiger Kohlenwasserstoffrest mit 1 bis 10 Kohlenstoffatomen je Rest,

wobei (Si)- die Bindung zum Siliciumatom der Siloxaneinheit der Formel (IV) bzw.(V)und -(N) die Bindung zum Stickstoffatom der Struktureinheit Y bzw. Y'der Formel (I) bzw. (I') bedeutet,

$R^5$ ein zweiwertiger Kohlenwasserstoffrest mit 1 bis 10 Kohlenstoffatomen je Rest ist, der durch ein Ethersauerstoffatom substituiert sein kann,

$R^6$ ein Wasserstoffatom oder ein einwertiger Kohlenwasserstoffrest mit 1 bis 10 Kohlenstoffatomen je Rest ist, der durch ein Ethersauerstoffatom substituiert sein kann, $R^7$ einen dreiwertigen Kohlenwasserstoffrest mit 3 bis 12 Kohlenstoffatomen je Rest ist und

t 0 oder 1 ist.

Beispiele für Reste $R^2$ sind aliphatische, cycloaliphatische und aromatenhaltige zweiwertige organische Reste, die Hydroxyfunktionen aus der Epoxidringöffnung enthalten, wie

$-CH(OH)CH_2-(N)$

$-(CH_2)_rCH(OH)CH_2-(N)$

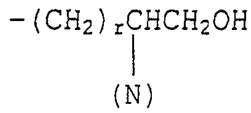

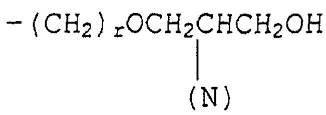

$-(CH_2)_rOCH_2CH(OH)CH_2-(N)$

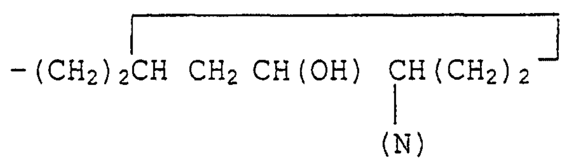

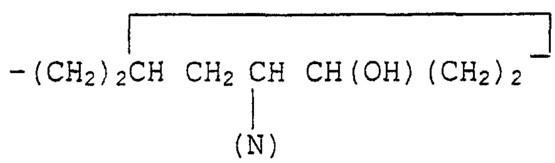

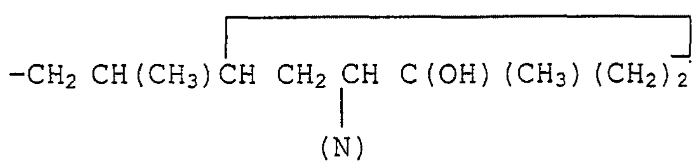

$-(CH_2)_2C_6H_4CH(OH)CH_2-(N)$

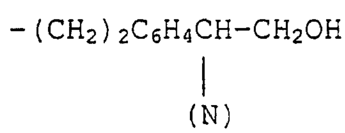

wobei r eine ganze Zahl von 1 bis 20, bevorzugt von 2 bis 8, ist, und -(N) die Bindung zum Stickstoffatom der Struktureinheit Y der Formel (I) bedeutet
und Alkylenreste, wie

$-(CH_2)-$

$-(CH_2)_2-$

$-(CH_2)_3-$

$-CH_2CH(CH_3)CH_2-$

$-CH_2CH_2CH(CH_3)-$

$-(CH_2)_4-$

$-(CH_2)_6-$

Bevorzugte Reste $R^2$ sind

$-(CH_2)_3OCH_2CH(OH)CH_2-(N)$

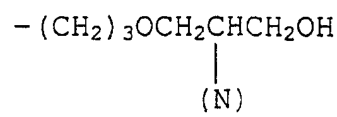

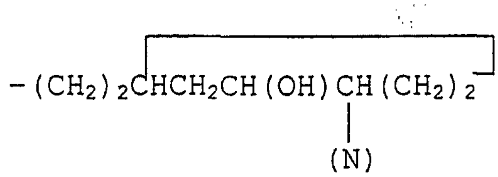

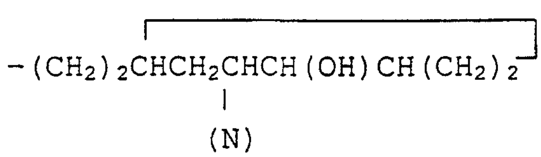

$-(CH_2)-$

$-(CH_2)_2-$

$-(CH_2)_3-$

$-CH_2CH(CH_3)CH_2-$

$-CH_2CH_2CH(CH_3)-$

$-(CH_2)_4-$

$-(CH_2)_6-$

wobei die beiden ersten Reste und der Rest $-(CH_2)_3-$ besonders bevorzugt sind.

Beispiele für Kohlenwasserstoffreste $R^3$ sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest, Cycloalkylreste, wie Cyclopentyl-, Cyclohexyl-, Cycloheptyl- und Methylcyclohexylreste; Arylreste, wie der Phenyl-, Naphthyl-, Anthryl- und Phenanthrylrest, Alkarylreste, wie o-, m-, p-Tolylreste, Xylylreste und Ethylphenylreste; und Aralkylreste, wie der Benzylrest, der $\alpha$- und der $\beta$-Phenylethylrest.

Beispiele für halogenierte Reste $R^3$ sind Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m- und p-Chlorphenylrest.

Beispiele für durch ein Stickstoffatom substituierte Reste $R^3$ sind

$-C_2H_4NEt_2$ bzw. $-C_2H_4N^+HEt_2 * X^-$

$-C_2H_4NMe_2$ bzw. $-C_2H_4N^+HMe_2 * X^-$

$-C_3H_6NMe_2$ bzw. $-C_3H_6N^+HMe_2 * X^-$

$-C_3H_6NEt_2$ bzw. $-C_3H_6N^+HEt_2 * X^-$

$-C_4H_8NMe_2$ bzw. $-C_4H_8N^+HMe_2 * X^-$

$-C_2H_4NMeC_2H_4NMe_2$ bzw. $-C_2H_4N^+HMeC_2H_4N^+HMe_2 * 2X^-$

$-C_3H_6NEtC_3H_6NEt_2$ bzw. $-C_3H_6N^+HEtC_3H_6N^+HEt_2 * 2X^-$

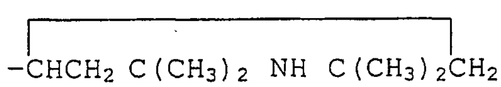 bzw. 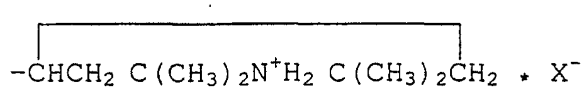

und

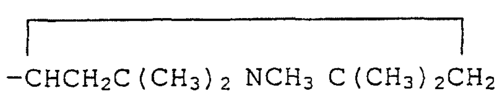 bzw. 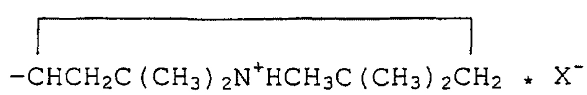

wobei Me ein Methylrest und Et ein Ethylrest bedeutet.

Beispiele für durch ein Sauerstoffatom substituierte Reste $R^3$ sind

$-(C_2H_4O)_sR$

$-(C_3H_6O)_sR$,

$-(C_2H_4O)_s(C_3H_6O)_sR$

und

$-(C_4H_8O)_sR$ ,

wobei s eine ganze Zahl von 1 bis 30, bevorzugt 1 bis 20, ist und R die oben dafür angegebene Bedeutung hat, bevorzugt ein Methyl- oder Butylrest bedeutet.

Beispiele für durch ein Stickstoff- und ein Sauerstoffatom substituierte Reste $R^3$ sind

$-(C_2H_4O)_sC_3H_6NR^1{}_2$ bzw. $-(C_2H_4O)_sC_3H_6N^+HR^1{}_2 * X^-$

$-(C_3H_6O)_sC_3H_6NR^1{}_2$ bzw. $-(C_3H_6O)_sC_3H_6N^+HR^1{}_2 * X^-$

und

$-(C_2H_4O)_s(C_3H_6O)_sC_3H_6NR^1{}_2$ bzw. $-(C_2H_4O)_s(C_3H_6O)_sC_3H_6N^+HR^1{}_2 * X^-$,

wobei s, $R^1$ und $X^-$ die oben dafür angegebene Bedeutung haben und $R^1$ bevorzugt ein Methyl- oder Ethylrest bedeutet.

Beispiele für durch ein Schwefelatom substituierte Reste $R^3$ sind

$-(C_2H_4S)_sR$

und

$-(C_3H_6S)_sR$ ,

wobei s und R die oben dafür angegebene Bedeutung haben und R bevorzugt ein Methyl-, Ethyl- oder Butylrest bedeutet.

Beispiele für Reste $R^4$ sind

$-(CH_2)_2-$

$-(CH_2)_3-$

$-CH_2CH(CH_3)-$

$-(CH_2)_4-$

$-CH_2C(CH_3)_2CH_2-$

$$-(CH_2)_6-$$

$$-(CH_2)_8-$$

$$-(CH_2)_{10}-$$

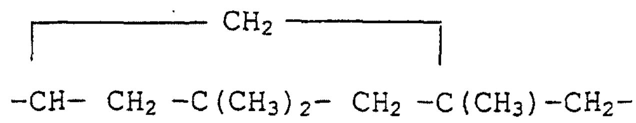

wobei Reste mit mindestens drei Kohlenstoffatomen bevorzugt sind und besonders bevorzugt die Reste

$-(CH_2)_3-$, $-(CH_2)_6-$, $-CH_2C(CH_3)_2CH_2-$ und

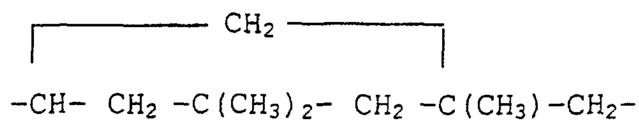

sind.

n ist vorzugsweise 0, 1, 2, 3, 4, 5, 6, 7, 8, 9 und 10.

Vorzugsweise werden die erfindungsgemäßen Ammoniumgruppen aufweisenden Organosiliciumverbindungen hergestellt durch Umsetzung von (Poly)aminen mit Epoxygruppen aufweisenden Organosiliciumverbindungen, insbesondere durch Polyaddition. Bevorzugt werden die durch Polyaddition erhaltenen Ammoniumgruppen aufweisenden Organosiliciumverbindungen hergestellt indem

in einer ersten Stufe

(Poly)amine (11) der allgemeinen Formel

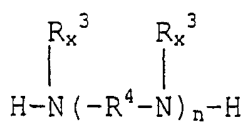

wobei $R^3$, $R^4$, n und x die oben dafür angegebene Bedeutung haben, mit Epoxygruppen aufweisenden Organosiliciumverbindungen (12) enthaltend Einheiten der allgemeinen Formel

$$E_cR_a(R^1O)_bSiO_{\frac{4-(a+b+c)}{2}}$$

wobei R, $R^1$, a und b die oben dafür angegebene Bedeutung haben, E gleich oder verschieden ist und einen einwertigen SiCgebundenen organischen Rest, der eine Epoxygruppe enthält, bedeutet und

c 0 oder 1 ist,

mit der Maßgabe, dass die Summe $a+b+c \leq 3$ ist und dass mindestens ein Rest E je Molekül enthalten ist,

umgesetzt werden,

mit der Maßgabe, dass das eingesetzte Verhältnis von N-gebundenem Wasserstoff in (Poly)amin (11) zu Epoxygruppe in Organosiliciumverbindung (12) ein solches ist, dass in Toluol lösliche, Aminogruppen aufweisende Organosiliciumverbindungen erhalten werden,

und in einer zweiten Stufe
die in der ersten Stufe erhaltenen Aminogruppen aufweisenden Organosiliciumverbindungen durch Zugabe von Säuren (14), vorzugsweise mit einem $pK_s$-Wert von größer als 0, bevorzugt größer als 2, besonders bevorzugt größer als 3, teilweise oder ganz protoniert werden, vorzugsweise ganz protoniert werden mit der Maßgabe, daß Ammoniumgruppen aufweisende Organosiliciumverbindungen erhalten werden, die mindestens eine Gruppierung aus zwei protonierten Stickstoffatomen, die über mindestens drei Kohlenstoffatome miteinander verbunden sind, enthalten.

Bei der Pclyaddition werden in der ersten Stufe als Aminogruppen aufweisende Organosiliciumverbindungen solche erhalten, die in Toluol löslich sind, d.h. es werden unvernetzte Organosiliciumverbindungen erhalten, im Gegensatz zu in Toluol unlöslichen Organosiliciumverbindungen, die vernetzt sind. Die erhaltenen Organosiliciumverbindungen sind in jedem Mischungsverhältnis in Toluol löslich, vorzugsweise sind sie bei einer Temperatur von 25°C und einem Druck von etwa bei 1020 hPa zu 100 Gewichtsprozent in Toluol löslich, wenn Organosiliciumverbindungen und Toluol im Verhältnis 1:1 (Gewichtsteile), bevorzugt 1:10 (Gewichtsteile), gemischt werden.

Die nach der Polyaddition erhaltenen Ammoniumgruppen aufweisenden Organosiliciumverbindungen enthalten Siloxanblöcke, die über mindestens einen zwei- oder mehrwertigen Ammoniumrest miteinander verbunden sind.

Beispiele für (Poly)amine (11) sind
primäre Amine der allgemeinen Formel $R^3$-$NH_2$,
wobei $R^3$ ein durch ein Stickstoffatom substituierter Rest ist, wie

$$Me_2NC_3H_6NH_2$$

$$Et_2NC_3H_6NH_2$$

$$Me_2NC_4H_8NH_2$$

$$Me_2NC_2H_4NMeC_2H_4NH_2$$

$$Et_2NC_3H_6NEtC_3H_6NH_2$$

4-Amino-2,2,6,6-tetramethylpiperidin,

und

4-Amino-1,2,2,6,6-pentamethylpiperidin,

und primäre Amine der allgemeinen Formel $R^3$-$NH_2$,
wobei $R^3$ ein durch ein Sauerstoff- und ein Stickstoffatom substituierter Rest ist, wie

$$Me_2NC_3H_6(C_2H_4O)_sC_3H_6NH_2$$

und

$$Et_2N(C_3H_6O)_s(C_2H_4O)_sC_3H_6NH_2,$$

wobei wobei s die oben dafür angegebene Bedeutung hat, Me ein Methylrest, Et ein Ethylrest und Bu ein n-Butylrest bedeutet.

Weitere Beispiele für (Poly)amine (11) sind Propylendiamin, 1,6-Diaminohexan, Dipropylentriamin, Isophorondiamin und Neopentandiamin.

Die Reste E sind vorzugsweise solche der Formel

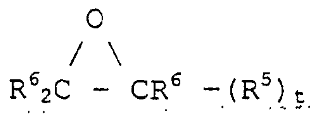

(VI´)

oder

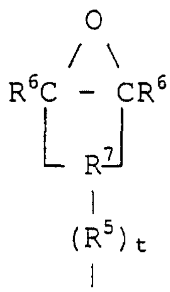

(VII´)

wobei $R^5$, $R^6$, $R^7$ und t die oben dafür angegebene Bedeutung haben.

Bei der Polyaddition werden bevorzugt als Epoxygruppen aufweisende Organosiliciumverbindungen (12) solche der allgemeinen Formel

$$E_dR_{3-d}SiO(SiR_2O)_o(SiREO)_pSiR_{3-d}E_d \qquad (VIII),$$

wobei R und E die oben dafür angegebene Bedeutung haben,
d 0 oder 1, insbesondere 1,
o 0 oder eine ganze Zahl von 1 bis 1000, insbesondere 5 bis 200, und
p 0 oder eine ganze Zahl von 1 bis 10, insbesondere 0 oder 1 bis 6, besonders bevorzugt 0, ist,
eingesetzt.

Die Epoxygruppen aufweisenden Organosiliciumverbindungen (12) haben vorzugsweise eine Viskosität von 1 bis 100.000 mPa.s bei 25°C, bevorzugt 10 bis 2.000 mPa.s bei 25°C.

Beispiele für Reste E sind
3,4-Epoxibutyl,
5,6-Epoxihexyl,
7,8-Epoxioctyl,
Glycidoxyethyl,
Glycidoxypropyl,
2-(3,4-Epoxicyclohexyl)ethyl,
2-(3-Epoxiphenyl)ethyl
sowie der Epoxirest selbst,
wobei der Glycidoxypropyl- und der 2-(3,4-Epoxicyclohexyl)ethyl bevorzugt sind, insbesonders der Glycidoxypropylrest.

Verfahren zur Herstellung von Epoxygruppen aufweisenden Organosiliciumverbindungen (12) sind dem Fachmann bekannt.

Bevorzugte Ausführungen sind die Epoxidation von aliphatisch ungesättigten Organopolysiloxanen und die durch Edelmetall(verbindungen) katalysierte Addition endständig ungesättigter organischer Epoxiverbindungen, wie Allylglycidether oder 4-Vinylcyclohexenoxyd an Organopolysiloxane, die Si-gebundenen Wasserstoff enthalten.

Die bei der Polyaddition eingesetzten Epoxygruppen aufweisenden Organosiliciumverbindungen (12) enthalten pro Molekül bevorzugt 1 bis 10, insbesondere 1 bis 6 Epoxygruppen. Eine besonders bevorzugte Ausführung ist die Verwendung von $\alpha,\omega$-Diepoxypolysiloxanen.

Bei der Polyaddition werden bevorzugt (Poly)amine (11) mit 2 bis 10 N-gebundenen Wasserstoffatomen, insbesondere mit 2 bis 6 N-gebundenen Wasserstoffatomen verwendet. Die Anzahl der Stickstoffatome pro Molekül ist davon zunächst unabhängig, beträgt aber bevorzugt 2 bis 4.

Bei der Polyaddition können gegebenenfalls Amine (13) mit nur einer N-H-Gruppe pro Molekül mitverwendet werden, da diese als Endstopper fungieren und so die Polyaddition kontrollierbarer machen.

Gegebenenfalls mitverwendete Amine (13) sind vorzugsweise solche der allgemeinen Formel

$$R^8-\underset{|}{\overset{H}{N}}-R^9 \quad (IX)$$

wobei $R^8$ und $R^9$ gleich oder verschieden sind und einen einwertigen Kohlenwasserstoffrest mit 1 bis 60 Kohlenstoffatomen je Rest bedeuten, der durch ein oder mehrere separate Heteroatome ausgewählt aus der Gruppe von Stickstoff- und Sauerstoffatomen unterbrochen sein kann, oder
$R^8$ und $R^9$ zusammen einen zweiwertigen Kohlenwasserstoffrest mit 4 bis 10 Kohlenstoffatomen bedeuten.
Beispiele für Amine (13) sind
Dibutylamin, Piperidin, Diethanolamin, Trimethylethylendiamin, Bis-(2-diethylaminoethyl)amin und Bis-(3-dimethylaminopropyl)amin.
Amine (13) werden gegebenenfalls vorzugsweise in Mengen von 5 bis 40 Gew.-% , bezogen auf das Gesamtgewicht der (Poly)amine (11) eingesetzt.
Bei der Polyaddition beträgt das Verhältnis von (Poly)aminen (11) zu Epoxygruppen aufweisenden Organosiliciumverbindungen (12) vorzugsweise 1:1 bis 10:1, bevorzugt 1:1 bis 5:1 und besonders bevorzugt 1:1 bis 4:1.
Bei der Polyaddition orientiert sich die Stöchiometrie der Reaktion am Verhältnis von N-gebundenem Wasserstoff in (11) zu Epoxygruppen in (12) (N-H/Epoxi). Dieses Verhältnis N-H/Epoxi kann in weiten Bereichen variiert werden, je nach Art der Einsatzstoffe und Zielbereich der Viskositäten der erfindungsgemäßen Organosiliciumverbindungen. Vorzugsweise wird aber ein N-H/Epoxi-Verhältnis von größer gleich ≥ 1 eingestellt, damit sämtliche Epoxygruppen abreagieren können unter der Bedingung, dass in Toluol lösliche Produkte, d.h. unvernetzte Produkte erhalten werden. Es liegt im Wissen des Fachmannes abhängig von der Anzahl der N-H-Gruppen in (11) und Epoxygruppen in (12), also der Funktionalität der Edukte, bei dem erfindungsgemäßen Verfahren das N-H/Epoxi-Verhältnis so einzustellen z.B. experimentell über das Durchführen von Versuchen, dass in Toluol lösliche Produkte erhalten werden. Da auch Nebenreaktionen sowie unvollständige Reaktionsfolgen mit Umsätzen unter 100 % der Theorie Einfluss nehmen, weiß der Fachmann, dass eventuelle Grenzwerte experimentell zu bestimmen sind, falls besonders viskose Produkte hergestellt werden sollen.
Die Pclyaddition wird in der ersten Stufe vorzugsweise bei Temperaturen über 25°C durchgeführt, obwohl auch bei normaler Umgebungstemperatur bereits eine nachweisbare Reaktion erfolgt. Im Interesse eines schnellen und vollständigen Reaktionsablaufes sind aber Temperaturen über 60°C bevorzugt, insbesonders im Bereich von 80 bis 180°C, besonders bevorzugt zwischen 100 und 150°C. Die Polyaddition wird vorzugsweise beim Druck der umgebenden Atmosphäre, also bei etwa 1000hPa, durchgeführt, wobei besonders bei flüchtigen (Poly)aminen (11) ein erhöhter Druck von Vorteil ist, um Verluste von N-H-Funktionen durch Abdampfen und damit eine Stöchiometrieänderung zu vermeiden.
Bei der Herstellung der erfindungsgemäßen Ammoniumgruppen aufweisenden Organosiliciumverbindungen werden vorzugsweise Säuren mit einem $pK_s$-Wert von größer als 0, bevorzugt größer als 2, besonders bevorzugt größer als 3, eingesetzt. Es kann eine Art von Säure oder verschiedene Arten von Säure eingesetzt werden. Vorzugsweise werden wasserlösliche organische oder anorganische Säuren eingesetzt.
Beispiele für Säuren sind
Monocarbonsäuren der allgemeinen Formel R'-COOH (Xa),
wobei R' ein Wasserstoffatom oder ein Kohlenwasserstoffrest mit 1 bis 18 Kohlenstoffatomen je Rest bedeutet, wie Ameisensäure, Essigsäure, Propionsäure, Buttersäure, Pivalinsäure, Sorbinsäure, Benzoesäure, Salicylsäure und Toluylsäure,
und Dicarbonsäuren der allgemeinen Formel HOOC-$R^5$-COOH (Xb),
wobei $R^5$ die oben dafür angegebene Bedeutung hat, wie Bernsteinsäure, Maleinsäure, Adipinsäure, Malonsäure und Phthalsäure,
wobei die Monocarbonsäuren bevorzugt sind.
Besonders bevorzugt sind Ameisensäure, Essigsäure und Propionsäure.
Weitere Beispiele für Säuren sind Sulfonsäuren der allgemeinen Formel R'-$SO_3H$ (Xc),
wobei R' die oben dafür angegebene Bedeutung hat, wie Methansulfonsäure, Butansulfonsäure, Trifluormethansulfonsäure und Toluolsulfonsäure,
sowie anorganische Säuren, wie Salzsäure, Bromwasserstoffsäure, Schwefelsäure und Phosphorsäure. Die ausschließliche Verwendung dieser starken Säuren ist nicht bevorzugt.
Beispiele für Kohlenwasserstoffreste R' sind die Kohlenwasserstoffreste R.
Dementsprechend enthalten die erfindungsgemäßen Organosiliciumverbindungen vorzugsweise die korrespondierenden Anionen $X^-$ der eingesetzten Säuren. Bevorzugt ist $X^-$ ein Anion einer korrespondierenden wasserlöslichen organischen oder anorganischen Säure. Beispiele für Anionen $X^-$ sind daher $R'-COO^-$ (Xa'), $^-OOC-R^5-COO^-$ (Xb') und $R'-SO_3^-$ (Xc') wobei R' und $R^5$ die oben dafür angegebene Bedeutung haben. Werden also die Aminogruppen aufweisenden Organosiliciumverbindungen beispielsweise mit Essigsäure umgesetzt, enthalten die erfindungsgemäßen Organosiliciumverbindungen die zu den protonierten Stickstoffatomen korrespondierenden Acetatanionen.

Die Säuren werden vorzugsweise in Mengen von 0,1 bis 2,0 Grammequivalent, bevorzugt von 0,5 bis 1,5 Grammequivalent, jeweils bezogen auf den Aminstickstoff der Aminogruppen aufweisenden Organosiliciumverbindungen, eingesetzt, wobei die Menge an Säure so bemessen wird, dass pro Molekül mindestens eine Gruppierung aus zwei protonierten Stickstoffatomen, die über mindestens drei Kohlenstoffatome miteinander verbunden sind, erhalten wird.

Enthält also 1 kg der Aminogruppen aufweisenden Organosiliciumverbindungen 14 g basischen Stickstoff, so werden beispielsweise vorzugsweise 6 bis 120 g Essigsäure, bevorzugt 30 bis 90 g Essigsäure verwendet. Der Einsatz von 6 bis 60 g Essigsäure führt in diesem Beispiel zu einer Teilprotonierung, d.h. nicht alle basischen Stickstoffatome der Aminogruppen aufweisenden Organosiliciumverbindungen werden protoniert. Bei Einsatz von 60 g Essigsäure und mehr werden vollprotonierte Produkte erhalten, wobei der Überschuß der Säure den pH-Wert der erfindungsgemäßen Ammoniumgruppen aufweisenden Organosiliciumverbindungen regelt. Durch noch höhere Säurezugabe kann der pH-Wert der erfindungsgemäßen Organosiliciumverbindungen zu noch tieferen Werten abgesenkt werden. Derartige Mischungen zeigen die Charakteristik gepufferter Systeme.

Bevorzugt werden alle basischen Stickstoffatome in den erfindungsgemäßen Organosiliciumverbindungen protoniert, wobei auch die gegebenenfalls in den Resten $R^3$ enthaltenen Stickstoffatome protoniert sind, so dass als

(a) Struktureinheit Y der Formel (I) bevorzugt solche der Formel

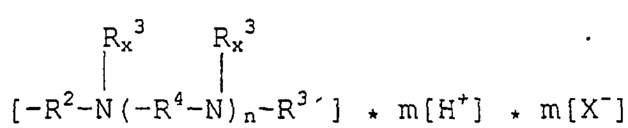

(I)

bzw. in anderer Schreibweise

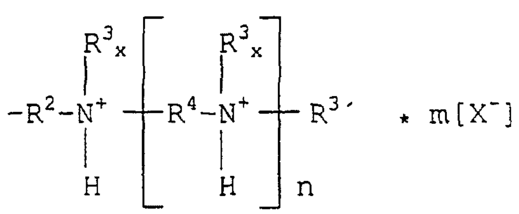

(Ia)

und als gegebenenfalls enthaltende
(d) Struktureinheit Y' der Formel (I') bevorzugt solche der Formel

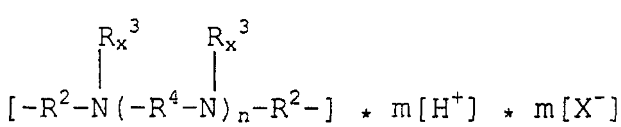

(I´)

bzw. in anderer Schreibweise

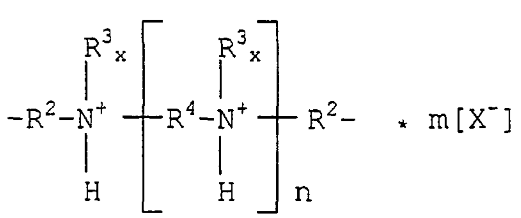

(Ia´)

wobei m gleich der Summe aus n+1 und der Gesamtanzahl aller in den Resten $R^3$ gegebenenfalls enthaltenen basischen Stickstoffatome ist,

[also höchstens m=n+1+(n+1)Σ(N-Atome in $R^3$)]

$R^2$, $R^3$, $R^{3'}$, $R^4$ und $X^-$ die oben dafür angegebene Bedeutung haben, mit der Maßgabe, dass die gegebenenfalls in $R^3$ enthaltenen basischen Stickstoffatome protoniert sind,

erhalten werden.

Die erfindungsgemäßen wässrigen Zusammensetzungen haben vorzugsweise einen Gehalt an Organosiliciumverbindung von 2 bis 60 Gew.-%, bevorzugt 2 bis 20 Gew.-%.

Die erfindungsgemäßen wässrigen Zusammensetzungen können auf Gebieten eingesetzt werden, wo auch bisher Ammoniumsiloxane verwendet werden, schwerpunktmäßig als Weichmacher für Substrate wie Fasern, Textilien, Haare; also polymerbasierende natürliche oder synthetische Substrate.

Zur Stabilisierung der erfindungsgemäßen wässrigen Zusammensetzungen können (4) mit Wasser mischbare Lösemittel, wie Isopropanol, Diethylenglykolmonomethylether, Diethylenglykolmonobutylether, Dipropylenglykol oder Dipropylenglykolmonomethylether mitverwendet werden.

Diese Lösemittel können vor oder nach der Zugabe von Säuren zugesetzt werden. Bevorzugt ist aber die Zugabe solcher Lösemittel vor einer Verdünnung mit Wasser.

Falls gewünscht, können auch (5) Emulgatoren zur Herstellung der wässriger Zusammensetzungen eingesetzt werden, wobei nichtionische bevorzugt sind.

Die erfindungsgemäßen wässrigen Zusammensetzungen haben den Vorteil, dass die erfindungsgemäßen Ammoniumgruppen aufweisenden Organosiliciumverbindungen bevorzugt in Wasser löslich oder selbstdispergierend sind, und dabei einen geringeren Gehalt an basischem Stickstoff aufweisen als die bisherigen Organosiliciumverbindungen. Insbesondere gilt dies im Vergleich zu Organosiliciumverbindungen, die Aminogruppen bzw. Ammoniumgruppen enthalten, in denen zwei Stickstoffatome ausschließlich durch ein oder zwei Kohlenstoffatome getrennt sind.

Gegenstand der Erfindung ist weiterhin ein Verfahren zur Imprägnierung von organischen Fasern mit den erfindungsgemäßen wässrigen Zusammensetzungen.

Nach dem erfindungsgemäßen Verfahren zur Imprägnierung von organischen Fasern können alle organischen Fasern in Form von Fäden, Garnen, Vliesen, Matten, Strängen, gewebten, gewirkten oder gestrickten Textilien imprägniert werden, die auch bisher mit Organosiliciumverbindungen imprägniert werden konnten. Beispiele für Fasern, die imprägniert werden können, sind solche aus Keratin, insbesondere Wolle, Mischpolymere von Vinylacetat, Baumwolle, Rayon, Hanf, natürliche Seide, Polypropylen, Polyethylen, Polyester, Polyurethan, Polyamid, Cellulose und Gemische aus mindestens zwei solcher Fasern. Wie aus der vorstehenden Aufzählung ersichtlich, können die Fasern natürlicher oder synthetischer Herkunft sein. Die Textilien können in Form von Stoffbahnen oder Kleidungsstücken bzw. Teilen von Kleidungsstücken vorliegen.

Das Auftragen auf die zu imprägnierenden Fasern kann in beliebiger für die Imprägnierung von Fasern geeigneter und vielfach bekannter Weise erfolgen, z. B. durch Tauchen, Streichen, Gießen, Sprühen, einschließlich Sprühen aus Aerosolverpackung, Aufwalzen, Klotzen oder Drucken.

Weiterhin können die erfindungsgemäßen wässrigen Zusammensetzungen auch in Formulierungen wie Cremes, Rasierschäumen, Shampoos, Waschlotionen, Seifen, Doedorants oder Haarsprays verwendet werden.

Ein weiterer Schwerpunkt ist die Verwendung zur Behandlung von mineralischen Stoffen, speziell zur Hydrophobierung von Oberflächen. Bevorzugt sind hierbei silikatische Oberflächen, auf denen die erfindungsgemäßen Organosiliciumverbindungen besonders gute Adhäsion zeigen. Gegenstand der Erfindung ist daher ein Verfahren zur Imprägnierung von silikatischen Oberflächen, insbesondere Glas, Keramik und Naturstein mit den erfindungsgemäßen wässrigen Zusammensetzungen.

Weiterhin können die erfindungsgemäßen wässrigen Zusammensetzungen zur Imprägnierung von flächigen organischen Kunststoffen, wie Vinylacetat, Polypropylen, Polyethylen, Polyester, Polyurethan, Polyamid und Polycarbonat verwendet werden. Gegenstand der Erfindung ist daher ein Verfahren zur Imprägnierung von flächigen organischen Kunststoffen mit den erfindungsgemäßen wässrigen Zusammensetzungen.

Beispiel 1:

197,8 g eines α,ω-Bis-(glycidyloxypropyl)-polydimethylsiloxans der durchschnittlichen Kettenlänge Si-51 werden mit 7,64 g Bis(3-aminopropyl)amin, 206 g Diethylenglykolmonobutylether und 5,8 g Isopropanol gemischt. Die dünnviskose Mischung von 23 $mm^2/s$ (25°C) wird auf 130°C erwärmt, worauf die Viskosität sehr stark ansteigt und nach 60 Minuten den Endwert von 10.100 $mm^2/s$ (25°C) erreicht. Nach dem Abkühlen auf 95°C werden 11,5 g Essigsäure eingerührt. Die Lösung enthält 0,41 equ. Aminstickstoff pro kg in protonierter Form (entspricht 0,57 Gew.-% protonierter Aminstickstoff).

30 g dieser Lösung werden mit 70 g Wasser verdünnt, wobei spontan eine pH-neutrale, klare, wässrige Formulierung entsteht, die beliebig weiter verdünnbar ist.

Beispiel 2:

250,0 g des Epoxisiloxans aus Beispiel 1 werden mit 10,66 g 1,6-Diaminohexan, 260,0 g Diethylenglykolmonobutylether und 7,5g Isopropanol abgemischt. Nach 2 Stunden bei 130°C erreicht die Reaktionsmischung eine Viskosität von 730 $mm^2/s$ (25°C). Beim Abkühlen wird 12,1 g Essigsäure zudosiert. Die Lösung enthält dann pro g 0,35 mequ. protonierten Aminstickstoff (entspricht 0,49 Gew.-% protonierter Aminstickstoff) und einen pH-Wert von ca. 7,5. Die weitere Abmischung mit der 2,3-fachen Wassermenge liefert eine kläre wässrige Formulierung.

Beispiel 3:

255,0 g des Epoxisiloxans aus Beispiel 1 wird mit 12,77 g Dimethylaminopropylamin und 7,5 g Isopropanol gemischt und wird ohne weiteres Lösemittel auf 120°C erwärmt. Nach insgesamt 6 Stunden ist die Viskosität nur auf den ca. 6-fachen Wert angestiegen (356 $mm^2/s$ bei 25°C). Das $^1H$-NMR-Spektrum dieser Probe zeigt einen Umsatz der Epoxidgruppen von über 99% der eingesetzten Menge. Das Reaktionsprodukt wird im Vakuum bei 120°C von flüchtigen Stoffen befreit. Es wird ein klares Öl mit einer Viskosität von 620 $mm^2/s$ bei 25°C erhalten.

30 g des basischen Aminosiloxancopolymers werden mit 1,4 g Essigsäure und 30 g Diethylenglykolmonobutylether gemischt und mit Wasser auf ein Gesamtgewicht von 200 g verdünnt. Die neutrale wässrige Formulierung enthält pro kg 106 mequ. protonierten Aminstickstoff (entspricht 0,15 Gew.-% protonierter Aminstickstoff). Beim weiteren Verdünnen entsteht keine Trübung.

Beispiel 4:

In einen mit Rührer, Rückflusskühler und Thermometer ausgestatteten 250 ml Dreihalskolben werden 100,0 g eines Polydimethylsiloxans mit Trimethylsilylendgruppen und Glycidyletherpropylseitengruppen mit einer Viskosität von 1.420 $mm^2/s$ bei 25°C und einem Epoxygehalt von 0,307 mMol/g, 6,1 g 3,3-Imino-bis(N,N-dimethylpropylamin) und 15,9 g 1-Butanol eingewogen und 6 Stunden bei ca. 135°C unter Rückfluss gerührt. Die leicht trübe, schwach gelbliche Lösung wird im Rotationsverdampfer bei 150°C und Vollvakuum ausgeheizt und anschließend filtriert. Das erhaltene Produkt mit einer Aminzahl von 0,91 mmol/g (entspricht 1,27 Gew.-% Aminstickstoff) ist schwach gelb gefärbt und klar.

15,0 g dieses Polymers werden in der gleichen Menge Diethylenglykolmonobutylether gelöst und mit 0,90 g Essigsäure protoniert. Unter kräftigem Rühren wird diese Mischung mit Wasser auf 100 g verdünnt. Es wird eine kläre wässrige Lösung erhalten, die ohne Ausfällungen weiter verdünnbar ist.

Beispiel 5:

54 g eines Hydrolysats aus 3-Aminopropyl-3-aminopropylmethyldimethoxysilan werden mit 1.090 g eines handelsüblichen Siliconöls mit Trimethylsiloxanendgruppen und einer Viskosität von 1000 $mm^2/s$ bei 25°C mit 0,35 g KOH gelöst und in 0,9 g Methanol bei 135°C equilibriert. Nach 5 Stunden befindet sich die Mischung im Gleichgewicht und der basische Katalysator wird mit 0,45 g Essigsäure neutralisiert. Nach dem Entfernen der flüchtigen Siloxancyclen im Vakuum bei 120°C werden 996 g eines Aminosiloxans mit $H_2N(CH_2)_3NH(CH_2)_3$-Gruppen erhalten.

30 g dieses Öls werden in der gleichen Menge Diethylenglykolmonobutylether gelöst und mit 1,2 g Essigsäure protoniert; dann wird mit Wasser auf 200 g ergänzt. Es wird eine klare wässrige Formulierung erhalten, die pro kg 90 mequ. protonierten Aminstickstoff (entspricht 0,13 Gew.-% protonierter Aminstickstoff) enthält.

## Patentansprüche

1. Wässrige Zusammensetzungen enthaltend

   (1) Ammoniumgruppen aufweisende Organosiliciumverbindungen, die pro Molekül mindestens eine Gruppierung der allgemeinen Formel

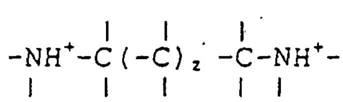

   wobei z eine ganze Zahl von 1 bis 20 bedeutet, enthalten

(2) Säureanionen, deren korrespondierende Säuren einen $pk_s$-Wert von größer als 0 haben,
(3) Wasser,
gegebenenfalls
(4) mit Wasser mischbare Lösemittel
und gegebenenfalls
(5) Emulgatoren.

2. Wässrige Zusammensetzungen nach Anspruch 1, **dadurch gekennzeichnet, dass** sie homogene wässrige Lösungen sind.

3. Wässrige Zusammensetzungen nach Anspruch 1 oder 2, **dadurch gekennzeichnet, dass** sie als (2) Säureanionen solche enthalten, deren korrespondierende Säuren einen $pk_s$-Wert von größer als 2 haben.

4. Wässrige Zusammensetzungen nach einem der Ansprüche 1 bis 3, **dadurch gekennzeichnet, dass** als. Ammoniumgruppen aufweisende Organosiliciumverbindungen solche enthaltend

(a') mindestens eine Siloxaneinheit der allgemeinen Formel

$$YR_a(R^1O)_bSiO_{\frac{4-(a+b)}{2}} \qquad (IV)$$

wobei R gleich oder verschieden ist und einen einwertigen gegebenenfalls halogenierten Kohlenwasserstoffrest mit 1 bis 18 Kohlenstoffatomen je Rest bedeutet,
$R^1$ gleich oder verschieden ist und einen einwertigen Kohlenwasserstoffrest mit 1 bis 8 Kohlenstoffatomen je Rest bedeutet,
a 0, 1, 2 oder 3 ist,
b 0, 1, 2 oder 3 ist,
mit der Maßgabe, dass die Summe a+b≤2 ist,
Y ein organischer Rest der allgemeinen Formel

$$[-R^2-\underset{\displaystyle R_x^3}{\overset{|}{N}}(-R^4-\underset{\displaystyle R_x^3}{\overset{|}{N}})_n-R^{3'}] \cdot m[H^+] \cdot m[X^-] \qquad (I)$$

bedeutet, wobei $R^2$ ein zweiwertiger organischer Rest ist, $R^3$ ein Wasserstoffatom oder einen einwertigen Kohlenwasserstoffrest mit 1 bis 60 Kohlenstoffatomen je Rest, der durch ein oder mehrere separate Heteroatome ausgewählt aus der Gruppe der Stickstoff-, Sauerstoff-, Schwefel- oder Halogenatome unterbrochen oder substituiert sein kann, bedeutet,
$R^{3'}$ die Bedeutung von $R^3$ hat,
$R^4$ ein zweiwertiger Kohlenwasserstoffrest mit 3 bis 10 Kohlenstoffatomen je Rest ist,
n 0 oder eine ganze Zahl von 1 bis 10 ist
m eine ganze Zahl von 2 bis zur Gesamtanzahl der Stickstoffatome in (I) ist
x gleich oder verschieden ist und 0 oder 1 ist,
$X^-$ ein Säureanion ist, deren korrespondierende Säure einen $pK_s$-Wert von größer als 0 hat,
mit der Maßgabe, daß die Struktureinheit der Formel (I) mindestens eine Gruppierung aus zwei protonierten Stickstoffatomen enthält, die über mindestens drei Kohlenstoffatome miteinander verbunden sind,
und
(b) mindestens eine Siloxaneinheit der allgemeinen Formel

$$R_a(R^1O)_bSiO_{\frac{4-(a+b)}{2}} \qquad (II)$$

wobei R, $R^1$, a und b die oben dafür angegebene Bedeutung haben, mit der Maßgabe, dass die Summe a+b≤3 ist, eingesetzt werden.

**5.** Wässrige Zusammensetzungen nach Anspruch 4, **dadurch gekennzeichnet, dass** $R^{3'}$ ein Wasserstoffatom ist.

**6.** Wässrige Zusammensetzungen nach Anspruch 4 oder 5, **dadurch gekennzeichnet, dass** die Ammoniumgrüppen aufweisenden Organosiliciumverbindungen

(d') mindestens eine Brückeneinheit der allgemeinen Formel

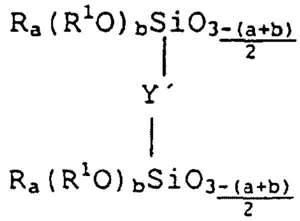

(V)

wobei R, $R^1$, a und b die im Anspruch 5 dafür angegebene Bedeutung haben, mit der Maßgabe, dass die Summe $a+b \leq 2$ ist, Y' ein organischer Rest der allgemeinen Formel

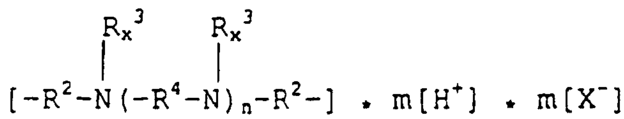

(I')

ist, wobei $R^2$, $R^3$, $R^4$, n, m, x und $X^-$ die im Anspruch 5 dafür angegebene Bedeutung haben,
mit der Maßgabe, daß die Struktureinheit der Formel (I') mindestens eine Gruppierung aus zwei protonierten Stickstoffatomen enthält, die über mindestens drei Kohlenstoffatome miteinander verbunden sind, enthalten.

**7.** Wässrige Zusammensetzungen nach Anspruch 4, 5 oder 6, **dadurch gekennzeichnet, dass**
$R^4$ ein Rest der Formel $-(CH_2)_3-$ ist.

**8.** Wässrige Zusammensetzungen nach einem der Ansprüche 4 bis
7, **dadurch gekennzeichnet, dass**
$R^2$ ein organischer Rest ausgewählt aus der Gruppe von

$$(Si)-(R^5)_t-CR^6(OH)-CR^6{}_2-(N) \qquad (VI),$$

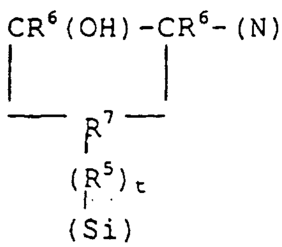

(VII)

und
ein zweiwertiger Kohlenwasserstoffrest mit 1 bis 10 Kohlenstoffatomen je Rest ist,
wobei (Si)- die Bindung zum Siliciumatom der Siloxaneinheit der Formel (IV) bzw. (V) und -(N) die Bindung zum Stickstoffatom des Restes Y bzw. Y' der Formel (I) bzw. (I') bedeutet,
$R^5$ ein zweiwertiger Kohlenwasserstoffrest mit 1 bis 10 Kohlenstoffatomen je Rest ist, der durch ein Ethersauerstoffatom substituiert sein kann,

$R^6$ ein Wasserstoffatom oder ein einwertiger Kohlenwasserstoffrest mit 1 bis 10 Kohlenstoffatomen je Rest ist, der durch ein Ethersauerstoffatom substituiert sein kann,
$R^7$ einen dreiwertigen Kohlenwasserstoffrest mit 3 bis 12 Kohlenstoffatomen je Rest ist und
t 0 oder 1 ist.

**9.** Wässrige Zusammensetzungen nach einem der Ansprüche 4 bis 8, **dadurch gekennzeichnet, dass** in den Ammoniumgruppen aufweisenden Organosiliciumverbinduhgen alle basischen Stickstoffatome protoniert sind und m gleich der Summe aus n+1 und der Gesamtanzahl aller in den Resten $R^3$ gegebenenfalls enthaltenen basischen Stickstoffatome ist.

**10.** Verfahren zur Herstellung der wässrigen Zusammensetzungen nach Anspruch 1, **dadurch gekennzeichnet, dass**

(1) Ammoniumgruppen aufweisende Organosiliciumverbindungen, die pro Molekül mindestens eine Gruppierung der allgemeinen Formel

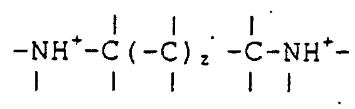

wobei z eine ganze Zahl von 1 bis 20 bedeutet, enthalten
(2) Säureanionen, deren korrespondierende Säure einen $pk_S$-Wert von größer als 2 haben, mit
(3) Wasser,
gegebenenfalls
(4) mit Wasser mischbare Lösemittel
und gegebenenfalls
(5) Emulgatoren
gemischt werden.

**11.** Verfahren zur Imprägnierung von organischen Fasern mit wässrigen Zusammensetzungen nach einem der Ansprüche 1 bis 9.

**12.** Verfahren zur Imprägnierung von silikatischen Oberflächen mit wässrigen Zusammensetzungen nach einem der Ansprüche 1 bis 9.

**13.** Verfahren zur Imprägnierung von flächigen organischen Kunststoffen mit wässrigen Zusammensetzungen nach einem der Ansprüche 1 bis 9.

## Revendications

**1.** Compositions aqueuses contenant

(1) des composés organosiliciés renfermant des groupes ammonium, qui comportent par molécule au moins un groupement de formule générale

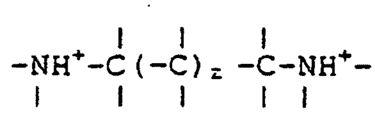

dans laquelle z représente un entier de 1 à 20,
(2) des anions d'acides, dont les acides correspondants présentent un $pk_S$ supérieur à 0,
(3) de l'eau,
éventuellement
(4) un solvant miscible à l'eau

et éventuellement
(5) des émulsifiants.

**2.** Compositions aqueuses selon la revendication 1, **caractérisées en ce qu'**elles sont des solutions aqueuses homogènes.

**3.** Compositions aqueuses selon la revendication 1 ou 2, **caractérisées en ce qu'**elles contiennent en tant que (2) des anions d'acides dont les acides correspondants présentent un $pk_s$ supérieur à 2.

**4.** Compositions aqueuses selon l'une quelconque des revendications 1 à 3, **caractérisées en ce que** l'on utilise en tant que composés organosiliciés renfermant des groupes ammonium, ceux comportant

(a') au moins un motif siloxane de formule générale

$$YR_a(R^1O)_bSiO_{\frac{4-(a+b)}{2}} \quad \text{(VI)}$$

dans laquelle R est identique ou différent et représente un radical hydrocarboné monovalent éventuellement halogéné, renfermant de 1 à 18 atomes de carbone par radical,
$R^1$ est identique ou différent et représente un radical hydrocarboné monovalent renfermant de 1 à 18 atomes de carbone par radical,
a vaut 0, 1, 2 or 3,
b vaut 0, 1, 2 or 3,
à condition que la somme a + b soit ≤ 2,
Y représente un radical organique de formule générale

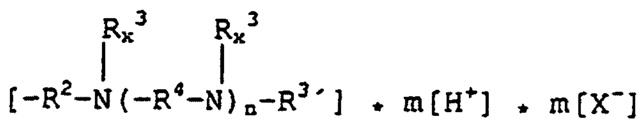 (I)

dans laquelle $R^2$ représente un radical organique bivalent,
$R^3$ représente un atome d'hydrogène ou un radical hydrocarboné monovalent renfermant de 1 à 60 atomes de carbone par radical, qui peut être interrompu ou substitué par un ou plusieurs hétéroatomes séparés choisis parmi le groupe constitué des atomes d'azote, d'oxygène, de soufre ou d'halogène,
$R^{3'}$ présente la signification de $R^3$,
$R^4$ représente un radical hydrocarboné bivalent ayant de 3 à 10 atomes de carbone par radical,
n vaut 0 ou est un entier de 1 à 10,
m est un entier de 2 jusqu'au nombre total d'atomes d'azote dans (I),
x est identique ou différent et vaut 0 ou 1,
$X^-$ représente un anion d'acide, dont l'acide correspondant présente un $pK_s$ supérieur à 0,
à condition que le motif structural de formule (I) renferme au moins un groupement de deux atomes d'azote protonés, qui sont liés l'un à l'autre par l'intermédiaire de trois atomes de carbone au moins, et
(b) au moins un motif siloxane de formule générale

$$R_a(R^1O)_bSiO_{\frac{4-(a+b)}{2}} \quad \text{(II)}$$

dans laquelle R, $R^1$, a et b présentent la signification indiquée ci-dessus à cet effet, à condition que la somme a + b soit ≤ 3.

**5.** Compositions aqueuses selon la revendication 4, **caractérisées en ce que** $R^{3'}$ représente un atome d'hydrogène.

**6.** Compositions aqueuses selon la revendication 4 ou 5, **caractérisées en ce que** les composés organosiliciés

renfermant des groupes ammonium comportent

(d') au moins un motif de pontage de formule générale

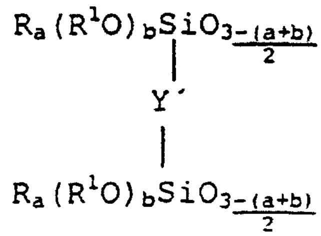

(V)

dans laquelle R, $R^1$, a et b présentent la signification indiquée dans la revendication 5 à cet effet, à condition que la somme a + b soit ≤ 2,
Y' représente un radical organique de formule générale

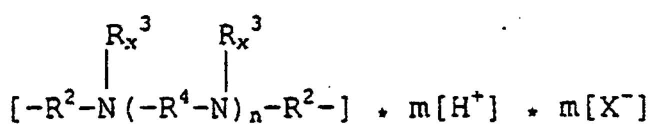

(I')

dans laquelle $R^2$, $R^3$, $R^4$, n, m, x et $X^-$ présentent la signification indiquée dans la revendication 5 à cet effet, à condition que le motif structural de formule (I') renferme au moins un groupement de deux atomes d'azote protonés, qui sont liés l'un à l'autre par l'intermédiaire de trois atomes de carbone au moins.

**7.** Compositions aqueuses selon la revendication 4, 5 ou 6, **caractérisées en ce que** $R^4$ représente un radical de formule $-(CH_2)_3-$.

**8.** Compositions aqueuses selon l'une quelconque des revendications 4 à 7, **caractérisées en ce que** $R^2$ représente un radical organique choisi parmi le groupe constitué de

$$(Si)-(R^5)_t-CR^6(OH)-CR^6{}_2-(N) \quad (VI),$$

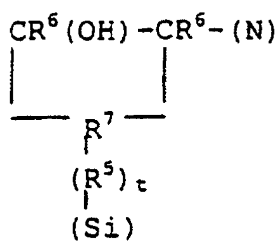

(VII)

et
d'un radical hydrocarboné bivalent renfermant de 1 à 10 atomes de carbone par radical,
où (Si)- représente la liaison à l'atome de silicium du motif siloxane de formule (IV) ou (V) et -(N) représente la liaison à l'atome d'azote du radical Y ou Y' de formule (I) ou (I'),
$R^5$ représente un radical hydrocarboné bivalent renfermant de 1 à 10 atomes de carbone par radical, qui peut être substitué par un atome d'oxygène d'éther,
$R^6$ représente un atome d'hydrogène ou un radical hydrocarboné monovalent renfermant de 1 à 10 atomes de

carbone par radical, qui peut être substitué par un atome d'oxygène d'éther,
$R^7$ représente un radical hydrocarboné trivalent renfermant de 3 à 12 atomes de carbone par radical, et
t vaut 0 ou 1.

**9.** Compositions aqueuses selon l'une quelconque des revendications 4 à 8, **caractérisées en ce que** dans les composés organosiliciés renfermant des groupes ammonium, tous les atomes d'azote basiques sont protonés et m est la somme de n + 1 et du nombre total de tous les atomes d'azote basiques éventuellement présents dans les radicaux $R^3$.

**10.** Procédé de préparation des compositions aqueuses selon la revendication 1, **caractérisé en ce que**

(1) des composés organosiliciés renfermant des groupes ammonium, qui comportent par molécule au moins un groupement de formule générale

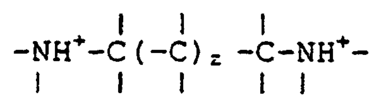

dans laquelle z représente un entier de 1 à 20,
(2) des anions d'acides, dont les acides correspondants présentent un $pk_s$ supérieur à 2,
(3) de l'eau,
éventuellement
(4) un solvant miscible à l'eau
et éventuellement
(5) des émulsifiants,
sont mélangés.

**11.** Procédé d'imprégnation de fibres organiques avec des compositions aqueuses selon l'une quelconque des revendications 1 à 9.

**12.** Procédé d'imprégnation de surfaces silicatées avec des compositions aqueuses selon l'une quelconque des revendications 1 à 9.

**13.** Procédé d'imprégnation de matières plastiques organiques plates avec des compositions aqueuses selon l'une quelconque des revendications 1 à 9.

## Claims

**1.** Aqueous compositions containing

(1) ammonio-containing organosilicon compounds which, per molecule, contain at least one moiety of the general formula

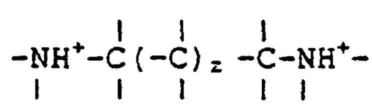

where z is an integer from 1 to 20,
(2) acid anions whose corresponding acids have a $pk_a$ value of greater than 0,
(3) water,
optionally
(4) water-miscible solvents
and optionally

(5) emulsifiers.

**2.** Aqueous compositions according to Claim 1, **characterized in that** they are homogeneous aqueous solutions.

**3.** Aqueous compositions according to Claim 1 or 2, **characterized in that** the corresponding acids of the acid anions (2) they contain have a $pk_a$ value of greater than 2.

**4.** Aqueous compositions according to any of Claims 1 to 3, **characterized in that** the ammonio-containing organosilicon compounds contain

(a') at least one siloxane unit of the general formula

$$YR_a(R^1O)_bSiO_{\frac{4-(a+b)}{2}} \quad (IV)$$

where each R is the same or different and represents a monovalent optionally halogenated hydrocarbon radical having 1 to 18 carbon atoms per radical,
each $R^1$ is the same or different and represents a monovalent hydrocarbon radical having 1 to 8 carbon atoms per radical,
a is 0, 1, 2 or 3,
b is 0, 1, 2 or 3,
with the proviso that the sum of a+b is ≤2,
Y is an organic radical of the general formula

$$[-R^2-\overset{\overset{R_x^3}{|}}{N}(-R^4-\overset{\overset{R_x^3}{|}}{N})_n-R^{3'}] \cdot m[H^+] \cdot m[X^-] \quad (I)$$

where $R^2$ is a divalent organic radical,
$R^3$ is a hydrogen atom or a monovalent hydrocarbon radical having 1 to 60 carbon atoms per radical which may be interrupted or substituted by one or more separate hetero atoms selected from the group consisting of nitrogen, oxygen, sulphur and halogen,
$R^{3'}$ has the meaning of $R^3$,
$R^4$ is a divalent hydrocarbon radical having 3 to 10 carbon atoms per radical,
n is 0 or an integer from 1 to 10,
m is an integer from 2 to the total number of nitrogen atoms in (I),
each x is the same or different and represents 0 or 1,
$X^-$ is an acid anion whose corresponding acid has a $pK_a$ value greater than 0,
with the proviso that the structural unit of formula (I) contains at least one moiety of two protonated nitrogen atoms which are joined together through at least three carbon atoms, and
(b) at least one siloxane unit of the general formula

$$R_a(R^1O)_bSiO_{\frac{4-(a+b)}{2}} \quad (II)$$

where R, $R^1$, a and b are each as defined above, with the proviso that the sum of a+b is ≤3.

**5.** Aqueous compositions according to Claim 4, **characterized in that** $R^{3'}$ is a hydrogen atom.

**6.** Aqueous compositions according to Claim 4 or 5, **characterized in that** the ammonio-containing organosilicon compounds contain

(d') at least one bridge unit of the general formula

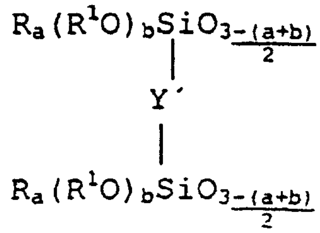

(V)

where R, $R^1$, a and b are each as defined in claim 5, with the proviso that the sum of a+b is $\leq 2$,
Y' is an organic radical of the general formula

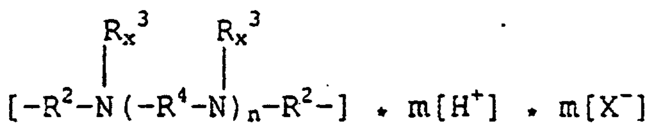

(I')

where $R^2$, $R^3$, $R^4$, n, m, x and $X^-$ are each as defined in Claim 5,

with the proviso that the structural unit of the formula (I') contains at least one moiety of two protonated nitrogen atoms which are joined together through at least three carbon atoms.

**7.** Aqueous compositions according to Claim 4, 5 or 6, **characterized in that** $R^4$ is a radical of the formula $-(CH_2)_3-$.

**8.** Aqueous compositions according to any of Claims 4 to 7, **characterized in that**
$R^2$ is an organic radical selected from the group consisting of

$$(Si) - (R^5)_t\text{-}CR^6(OH)\text{-}CR^6{}_2\text{-}(N) \qquad (VI),$$

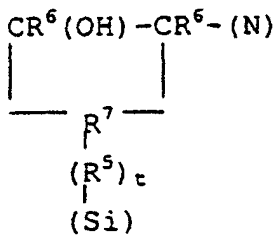

(VII)

and
a divalent hydrocarbon radical having 1 to 10 carbon atoms per radical,
where (Si)- is the bond to the silicon atom of the siloxane unit of the formula (IV) or (V) as the case may be and -(N) is the bond to the nitrogen atom of the radical Y or Y' of the formula (I) or (I') as the case may be,
$R^5$ is a divalent hydrocarbon radical having 1 to 10 carbon atoms per radical which may be substituted by an ether oxygen atom,
$R^6$ is a hydrogen atom or a monovalent hydrocarbon radical having 1 to 10 carbon atoms per radical which may be substituted by an ether oxygen atom,
$R^7$ is a trivalent hydrocarbon radical having 3 to 12 carbon atoms per radical and
t is 0 or 1.

**9.** Aqueous compositions according to any of Claims 4 to 8, **characterized in that** all the basic nitrogen atoms in the ammonio-containing organosilicon compounds are protonated and m is equal to the sum formed from n+1 and the total number of any basic nitrogen atoms contained in the $R^3$ radicals.

**10.** Process for preparing the aqueous compositions of Claim 1, **characterized in that** it comprises mixing

(1) ammonio-containing organosilicon compounds which, per molecule, contain at least one moiety of the general formula

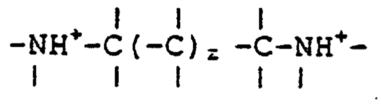

where z is an integer from 1 to 20,
(2) acid anions whose corresponding acids have a $pk_a$ value of greater than 2,
(3) water,
optionally
(4) water-miscible solvents
and optionally
(5) emulsifiers.

**11.** Process for impregnating organic fibres with aqueous compositions according to any of Claims 1 to 9.

**12.** Process for impregnating silicatic surfaces with aqueous compositions according to any of Claims 1 to 9.

**13.** Process for impregnating sheetlike organic plastics with aqueous compositions according to any of Claims 1 to 9.